# EUROPÄISCHE PATENTSCHRIFT

(11) **EP 0 352 251 B1**
(45) Veröffentlichungstag und Bekanntmachung des Hinweises auf die Patenterteilung: **13.01.1993**
(21) Anmeldenummer: 89890191.3
(22) Anmeldetag: 18.07.1989
(51) Int. Cl.: A61F 2/54

(54) **Künstliche Hand**
Artificial hand
Main artificielle

(30) Priorität: 18.07.1988 AT 1841/88
(43) Veröffentlichungstag der Anmeldung: 24.01.1990
(73) Patentinhaber: Otto Bock Orthopädische Industrie Besitz- und Verwaltungs-Kommanditgesellschaft, 37115 Duderstadt (DE)
(72) Erfinder: Horvath, Eduard, A-1070 Wien (AT)
(74) Vertreter: Casati, Wilhelm, Dipl.-Ing.

(56) Entgegenhaltungen:
- US-A- 2 494 460
- US-A- 2 629 107

## Beschreibung

Die Erfindung bezieht sich auf eine mit einem Prothesenanschluß versehene Handprothese, welche einen dem Daumen entsprechenden ersten Greifer und mindestens einen weiteren, wenigstens einem anderen Finger der Hand entsprechenden zweiten Greifer besitzt, wobei der erste und zweite Greifer gegeneinander verschwenkbar angeordnet sind.

Bei den bekannten Ausbildungen dieser Art (siehe z.B. US-A-2 629 107) sind die Greifer um quer zum Prothesenanschluß verlaufende Achsen schwenkbar, wobei sie wie Zangen funktionieren, mit welchen durch das Zueinanderbewegen der Backen ein Gegenstand angegriffen werden kann. Diese Ausbildungen haben den Nachteil, daß sie, insbesondere beim Aufheben von Gegenständen von höherliegenden Flächen, sehr schwer in Greifposition bringbar sind, da der Arm in entsprechendem Winkel zu jener Fläche gehalten werden muß, auf welcher der aufzuhebende Gegenstand aufruht.

Der Erfindung liegt die Aufgabe zugrunde, eine künstliche Hand der eingangs genannten Art zu schaffen, welche einen möglichst natürlichen Greifvorgang ermöglicht.

Erfindungsgemäß wird diese Aufgabe dadurch gelöst, daß der erste Greifer unter einem stumpfen Winkel von dem ihm zugeordneten Daumenträger und der zweite Greifer ebenfalls unter einem stumpfen Winkel von dem ihm zugeordneten Fingerträger wegragt, daß der Daumenträger und der Fingerträger zur gegenseitigen Verschwenkung der Greifer zueinander um eine Achse verschwenkbar gelagert sind, die einen spitzen Winkel mit der auf die Achse des Prothesenanschlusses normal stehenden Ebene einschließt, und daß der Antrieb zur Schwenkbewegung der Greifer gegebenenfalls im Fingerträger und/oder Daumenträger angeordnet ist, welche Träger dann zur Aufnahme des Antriebes hülsenförmig gestaltet sind. Dadurch ist es möglich, der natürlichen Greifbewegung einer Hand entsprechend einen Gegenstand von einer Ebene aufzuheben, wobei die Haltung des gesamten Armes ebenfalls der natürlichen Haltung entspricht. Durch die hülsenförmige Gestaltung der Fingerträger und/oder der Daumenträger ist es möglich, den Antrieb in die Hülsen einzubauen, wodurch eine raumsparende Ausführung erreicht wird, die es erlaubt, eine auch vom kosmetischen Standpunkt ansprechende Kunsthand zu schaffen.

Vorteilhafterweise kann der Winkel zwischen der Schwenkachse des jeweils schwenkbaren Trägers und der Normalebene auf die Achse des Prothesenanschlusses zwischen 30° und 70° betragen. Durch diese Neigung der Drehachse wird die günstigste Haltung im Hinblick auf eine natürliche Hand erzielt. Dabei kann das Schwenklager des Fingerträgers auf einem Arm angeordnet sein, der mit dem Prothesenanschluß in Verbindung steht. Dadurch erfolgt die Bewegung der den Zeigefinger und den Mittelfinger bildenden Greifer entsprechend den Fingern einer natürlichen Hand.

Bei einer besonders bevorzugten Ausbildung kann der das Schwenklager des Fingerträgers aufweisende Arm mit dem Prothesenanschluß schwenkbar verbunden sein, wobei die Schwenkachse des Armes in einem am Prothesenanschluß vorgesehenen Lagerträger parallel zur Schwenkachse des Fingerträgers verläuft und bevorzugt mit der Schwenkachse des dem Daumen entsprechenden Greifers zusammenfallt. Bei dieser Ausuhrung gelingt es, aufgrund der Schwenkbarkeit des Armes unangenehmen Verzerrungen der kosmetischen Verkleidung vorzubeugen. Für eine gleichzeitige Gegeneinanderbewegung der den Zeigefinger und den Mittelfinger bildenden Greifer und dem dem Daumen entsprechenden Greifer ist es günstig, wenn die Schwenkachse des Armes mit der Schwenkachse des dem Daumen entsprechenden Greifers zusammenfällt. Für einen besonders guten Ausgleich der Bewegung zur Schonung des kosmetischen Überzuges über die künstliche Hand kann der Fingerträger über eine Lasche mit dem Prothesenanschluß, bevorzugt mit dem Lagerträger, gekoppelt sein. Dadurch entsteht ein Gelenksviereck, das eine gesteuerte Bewegung des Lagerträgers um den im Prothesenanschluß gelagerten Arm erlaubt.

Bei einer einfacheren Ausführungsvariante kann der das Schwenklager des Fingerträgers aufweisende Arm starr mit dem Prothesenanschluß verbunden sein, wobei gegebenenfalls der dem Daumen entsprechende Greifer starr am Prothesenanschluß gehalten ist. Es kann jedoch auch der Fingerträger mit dem Prothesenanschluß fest verbunden sein, wobei der dem Daumen entsprechende Greifer schwenkbar gelagert ist.

Mit beiden Ausuhrungsvarianten ist wohl ein leichtes Eingreifen eines auf einer Fläche liegenden Gegenstandes ermöglicht, jedoch wird dabei nur einer der beiden gegeneinander bewegbaren Greifer verschwenkt, was die Greifbewegung etwas erschweren kann und etwas ungünstige Auswirkungen hinsichtlich der Verformung des kosmetischen Überzuges der Hand zeitigt.

In der Zeichnung sind zwei Ausführungsbeispiele des Erfindungsgegenstandes dargestellt.

Fig. 1 zeigt eine Seitenansicht eines ersten Ausführungsbeispieles der erfindungsgemäßen künstlichen Hand.

Fig. 2 zeigt eine Draufsicht auf dieselbe.

Fig. 3 gibt einen Schnitt nach Linie III-III der Fig. 2, in Richtung der Achse des Fingerträgers gesehen, wieder.

Fig. 4 veranschaulicht eine Vorderansicht der erfindungsgemäßen künstlichen Hand in Richtung der Achse des Prothesenanschlusses gesehen.

Fig. 5 ist eine Ansicht eines zweiten Ausführungsbeispieles in Richtung der Achse des Fingerträgers gesehen.

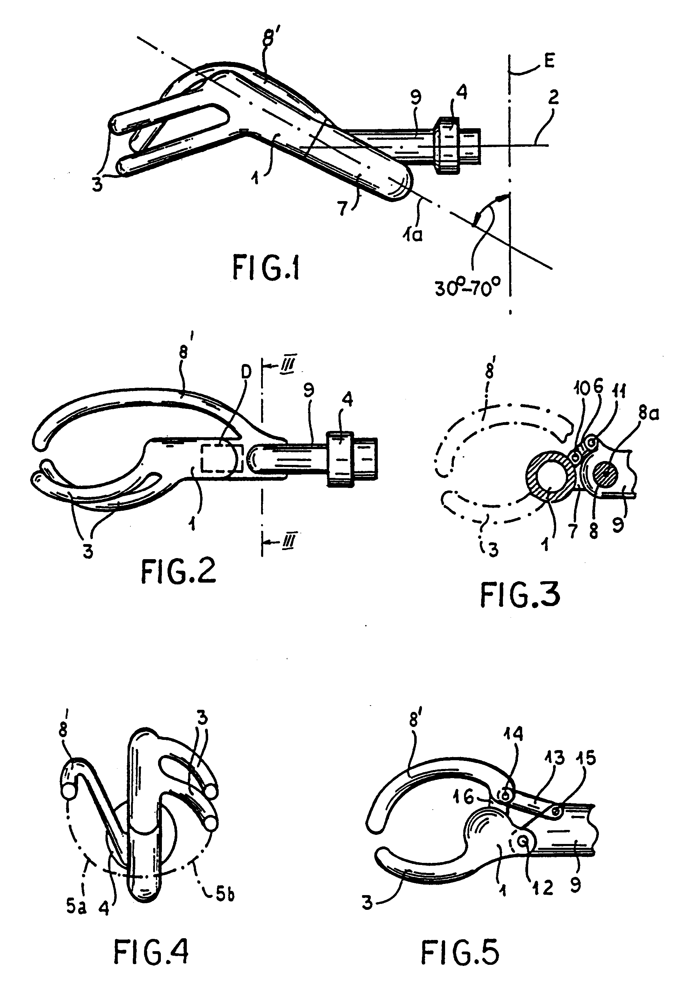

An einem Fingerträger 1 sind unter einem stumpfen Winkel wegragend Greifer 3 angebracht, welche fingerartig gekrümmt sind. Diese beiden Greifer 3 entsprechen ungefähr dem Zeigefinger und dem Mittelfinger einer natürlichen Hand. Der Fingerträger 1 ist an einem Arm 7 schwenkbar gelagert, welcher von einem Prothesenanschluß 4 seitlich wegragt. Die Drehachse 1a des Fingerträger 1 schließt dabei zur Normalachse E auf die Achse 2 des Prothesenanschlusses 4 einen Winkel von etwa 30° bis 70° ein, wobei sich der Winkel danach richtet, wie die natürliche Hand des Prothesenträgers ausgebildet ist. Durch die Wahl des entsprechenden Winkels kann die natürliche Handform des Prothesenträgers entsprechend nachgeahmt werden. Der seitlich wegragende Arm 7 ist an einem Lagerträger 9 schwenkbar gelagert, und zwar über einen Bolzen 8. Dieser Bolzen 8 bzw. diese Achse 8a bildet den Daumenträger und trägt in Fortsetzung den dem Daumen entsprechenden Greifer 8′. Wie aus Fig. 3 ersichtlich, weist sowohl der Fingerträger 1 als auch der Lagerträger 9 einen seitlichen Fortsatz 10 bzw. 11 auf, in welchem eine Lasche 6 schwenkbar gelagert ist. Diese Lasche 6 bildet somit mit den übrigen Teilen ein Gelenksviereck, bei welchem durch den seitlich wegragenden Arm 7 eine Kurbel des Gelenksviereckes, durch den Fingerträger 1 die Koppel, durch den Prothesenanschluß 4 das Gestell und durch die Lasche 6 die zweite Kurbel gebildet ist.

Der Antrieb für die Bewegung der Greifer ist zwischen dem Fingerträger 1 und dem seitlich wegragenden Arm 7 angeordnet und ist in vorliegendem Fall nicht dargestellt. Es wird dabei ein herkömmlicher motorischer Antrieb innerhalb der beiden Teile angeordnet, die hülsenförmig gestaltet sind.

Bei Einschalten des Bewegungsantriebes wird einerseits der Fingerträger 1 in bezug auf den seitlichen Arm 7, und über die Lasche 6 gleichzeitig auch der den Daumen bildende Greifer 8′ über die Achse 8 im Lagerträger 9 verschwenkt. Die beiden Schwenkbahnen sind in Fig. 4 wiedergegeben, und zwar die Schwenkbahn des den Daumen bildenden Greifers 8′ durch die Bahn 5a und die Schwenkbahn der die Finger bildenden Greifer 3 durch den strichpunktierten Weg 5b.

Beim Ausführungsbeispiel gemäß Fig. 5 ist der Fingerträger 1 am Lagerträger 9 direkt über das Schwenklager 12 gelagert. Der Daumen 8′ ist über einen Winkelhebel 16 mit dem Antrieb, welcher innerhalb des Fingerträgers 1 angeordnet ist, direkt verbunden und bildet somit das Gegenlager für den Antrieb des Fingerträgers 1. Am Winkelhebel 16 greift bei 14 eine Lasche 13 schwenkbar an, welche mit ihrem anderen Ende bei 15 ihrerseits am Lagerträger 9, angelenkt ist. Es wird damit wieder ein Gelenksviereck gebildet, wobei der Fingerträger 1 mit seinem zum Lager 12 reichenden Teil das Gestell bildet, der Bereich des Lagerträgers 9 zwischen 12 und 15 die eine Kurbel, der Hebel 16 die andere Kurbel und die Lasche 13 die Koppel bildet.

Wird nun durch den Antrieb der Fingerträger 1, in Fig. 5 gesehen, gegen den Uhrzeigersinn verschwenkt, dann wird gleichzeitig der Winkelhebel 16 unter Verschwenken des Greifers 8′ im Uhrzeigersinn bewegt, wobei die Bewegung des Fingerträgers 1 um den Anlenkpunkt 12 und die Bewegung des Winkelhebels 16 um den Anlenkpunkt 14 erfolgt, wobei sich der Anlenkpunkt 14 um den Punkt 15 der Koppel 13 frei schwenken kann. Dadurch wird ein Öffnen der beiden Greifer 3 und 8′ erzielt. Beim Schließen der Hand erfolgt die Bewegung des Fingerträgers 1 in entgegengesetzter Richtung, d.h. also im Uhrzeigersinn, wodurch ein Schwenken des Winkelhebels 16 und damit des Greifers 8′ entgegen dem Uhrzeigersinn erfolgt, was ein Schließen der beiden Greifer bewirkt.

Bei den einfachen Ausführungsvarianten, bei welchen einer der beiden Greiferteile feststehend angeordnet ist, wird man den jeweiligen Greifer, also entweder den Greifer 8′ oder die Greifer 3, am Ende der Bewegungsbahn, also etwa in dem Berührungsbereich der Bahnen 5a und 5b, feststehend anordnen, wobei der andere Teil dann über den halben Bereich, also entweder die Bahn 5a oder die Bahn 5b, bewegt wird.

## Patentansprüche

1. Mit einem Prothesenanschluß versehene Handprothese, welche einen dem Daumen entsprechenden ersten Greifer und mindestens einen weiteren, wenigstens einem anderen Finger der Hand entsprechenden zweiten Greifer besitzt, wobei der erste und zweite Greifer gegeneinander verschwenkbar angeordnet sind, dadurch gekennzeichnet, daß der erste Greifer (8′) unter einem stumpfen Winkel von dem ihm zugeordneten Daumenträger (8) und der zweite Greifer (3) ebenfalls unter einem stumpfen Winkel von dem ihm zugeordneten Fingerträger (1) wegragt, daß der Daumenträger (8) und der Fingerträger (1) zur gegenseitigen Verschwenkung der Greifer (8′, 3) zweinander um eine Achse (A) verschwenkbar gelagert sind, die einen spitzen Winkel mit der auf die Achse (2) des Prothesenanschlusses (4) normal stehenden Ebene (E) einschließt, und daß der Antrieb zur Schwenkbewegung der Greifer (8′, 3) gegebenenfalls im Fingerträger (1) und/oder Daumenträger (8) angeordnet ist, welche Träger dann zur Aufnahme des Antriebes hülsenförmig gestaltet sind.

2. Künstliche Hand nach Anspruch 1, dadurch gekennzeichnet, daß der Winkel zwischen der Schwenkachse des jeweils schwenkbaren Trägers und der Normalebene (E) auf die Achse (2) des Prothesenanschlusses (4) zwischen 30° und 70° beträgt.

3. Künstliche Hand nach Anspruch 1 oder 2, dadurch gekennzeichnet, daß das Schwenklager des Fingerträgers (1) auf einem Arm (7) angeordnet ist, der mit dem Prothesenanschluß (4) in Verbindung steht.

4. Künstliche Hand nach Anspruch 3, dadurch gekennzeichnet, daß der das Schwenklager des Fingerträgers (1) aufweisende Arm (7) mit dem Prothesenanschluß (4) schwenkbar verbunden ist, wobei die Schwenkachse (8) des Armes (7) in einem am Prothesenanschluß (4) vorgesehenen Lagerträger (9) parallel zur Schwenkachse des Fingerträgers (1) verläuft und bevorzugt mit der Schwenkachse des dem Daumen entsprechenden Greifers (8′) zusammenfällt.

5. Künstliche Hand nach Anspruch 4, dadurch gekennzeichnet, daß der Fingerträger (1) über eine Lasche (6) mit dem Prothesenanschluß, bevorzugt mit dem Lagerträger (9) gekoppelt ist.

6. Künstliche Hand nach einem der Ansprüche 1 bis 3, dadurch gekennzeichnet, daß der das Schwenklager des Fingerträgers (1) aufweisende Arm (7) starr mit dem Prothesenanschluß (4) verbunden ist, wobei gegebenenfalls der dem Daumen entsprechende Greifer (8′) starr am Prothesenanschluß (4) gehalten ist.

7. Künstliche Hand nach einem der Ansprüche 1 bis 3, dadurch gekennzeichnet, daß der Fingerträger (1) mit dem Prothesenanschluß (4) fest verbunden ist, wobei der dem Daumen entsprechende Greifer (8′) schwenkbar gelagert ist.

## Claims

1. Hand prosthesis, provided with a prosthesis connection, which possesses a first claw corresponding to the thumb and at least one further second claw corresponding to at least one other finger of the hand, the first and second claws being counter-pivotably disposed, characterised in that the first claw (8′) projects at an obtuse angle from its assigned thumb carrier (8) and the second claw (3) likewise projects at an obtuse angle from its assigned finger carrier (1), in that the thumb carrier (8) and the finger carrier (1), for the reciprocal relative pivoting of the claws (8′, 3), are mounted pivotably about an axis (A) forming an acute angle with the plane (E) lying perpendicular to the axis (2) of the prosthesis connection (4), and in that the drive mechanism for the swivel movement of the claws (8′, 3) is disposed, where appropriate, in the finger carrier (1) and/or thumb carrier (8), which carriers are in this case designed in the shape of a sleeve for receiving the drive mechanism.

2. Artificial hand according to Claim 1, characterised in that the angle between the swivel axis of the respectively pivotable carrier and the plane (E) perpendicular to the axis (2) of the prosthesis connection (4) measures between 30° and 70°.

3. Artificial hand according to Claim 1 or 2, characterised in that the swivel bearing of the finger carrier (1) is disposed on an arm (7), which is joined to the prosthesis connection (4).

4. Artificial hand according to Claim 3, characterised in that the arm (7) exhibiting the swivel bearing of the finger carrier (1) is pivotably joined to the prosthesis connection (4), the swivel axis (8) of the arm (7) running, in a bearing carrier (9) provided on the prosthesis connection (4), parallel to the swivel axis of the finger carrier (1) and preferably coinciding with the swivel axis of the claw (8′) corresponding to the thumb.

5. Artificial hand according to Claim 4, characterised in that the finger carrier (1) is coupled by means of a tongue (6) to the prosthesis connection, preferably to the bearing carrier (9).

6. Artificial hand according to one of Claims 1 to 3, characterised in that the arm (7) exhibiting the swivel bearing of the finger carrier (1) is rigidly joined to the prosthesis connection (4), the claw (8′) corresponding to the thumb being rigidly secured, where appropriate, to the prosthesis connection (4).

7. Artificial hand according to one of Claims 1 to 3, characterised in that the finger carrier (1) is fixed to the prosthesis connection (4), the claw (8′) corresponding to the thumb being pivotably mounted.

## Revendications

1. Prothèse de main, pourvue d'un raccord, possédant une première griffe correspondant au pouce et au moins une autre seconde griffe, correspondant au moins à un autre doigt de la main, dans laquelle la première et la seconde griffes peuvent pivoter l'une par rapport à l'autre, caractérisée en ce que la première griffe (8′) s'éloigne sous un angle obtus du support de pouce (8) qui lui est associé et la seconde griffe (3) s'éloigne également sous un angle obtus du support de doigt (1) qui lui est associé, en ce que le support de pouce (8) et le support de doigt (1) sont montés, en vue du pivotement réciproque des griffes (8′, 3), de manière a pivoter l'un par rapport à l'autre autour d'un axe (A) qui forme un angle aigu avec le plan (E) normal a l'axe (2) du raccord (4) de la prothèse et en ce que le mécanisme d'entraînement en vue du pivotement des griffes (8′, 3) est également monté dans le support de doigt (1) et/ou le support de pouce (8), ces supports ayant la forme d'un manchon destiné à loger le mécanisme d'entraînement.

2. Main artificielle selon la revendication 1, caractérisée en ce que l'angle compris entre l'axe de pivotement du support pivotant respectif et le plan (E) normal à l'axe (2) du raccord (4) de la prothèse, est compris entre 30° et 70°.

3. Main artificielle selon la revendication 1 ou 2, caractérisée en ce que le pivot du support de doigt (1) se situe sur un bras (7) qui est relié au raccord de prothèse (4).

4. Main artificielle selon la revendication 3, caractérisée en ce que le bras (7) comportant le pivot du support de doigt (1) est assemblé pivotant avec le raccord de prothèse (4), l'axe de pivotement (8) du bras (7) s'étendant dans un porte-pivot (9) prévu sur le raccord de prothèse (4), parallèlement à l'axe de pivotement du support de doigt (1) et coïncidant de préférence avec l'axe de pivotement de la griffe (8′) correspondant au pouce.

5. Main artificielle selon la revendication 4, caractérisée en ce que le support de doigt (1) est accouplé avec le raccord de prothèse, de préférence avec le porte-pivot (9), par une éclisse (6).

6. Main artificielle selon l'une des revendications 1 à 3, caractérisée en ce que le bras (7), présentant le pivot du support de doigt (1), est assemblé rigidement avec le raccord de prothèse (4), la griffe (8′), correspondant au pouce, étant éventuellement maintenue rigidement sur le raccord de prothèse (4).

7. Main artificielle selon l'une des revendications 1 à 3, caractérisée en ce que le support de doigt (1) est assemblé de manière fixe au raccord de prothèse (4), la griffe (8′) correspondant au pouce étant montée de manière à pouvoir pivoter.
